# EUROPEAN PATENT SPECIFICATION

(11) **EP 2 830 588 B1**
(45) Date of publication and mention of the grant of the patent: **10.05.2017**
(21) Application number: 13722550.4
(22) Date of filing: 27.03.2013
(51) Int. Cl.: A61K 8/99, A61Q 19/00

(54) **USE OF A LYSATE OF A CULTURE OF A BACTERIUM OF THE VITREOSCILLA SP. GENUS FOR PREVENTING AND/OR TREATING HYPERSEBORRHOEIC CONDITIONS OF THE SCALP**
VERWENDUNG EINES LYSATS EINER BAKTERIENKULTUR VON VITREOSCILLA SP. GATTUNG ZUR PRÄVENTION UND/ODER BEHANDLUNG VON HYPERSEBORRHOISCHER KOPFHAUT
UTILISATION D'UN LYSAT D'UNE CULTURE DE BACTÉRIE DU GENRE VITREOSCILLASP. DANS LA PRÉVENTION ET/OU LE TRAITEMENT D'ÉTATS D'HYPERSÉBORRHÉE DU CUIR CHEVELU

(30) Priority: 27.03.2012 FR 1252742
(43) Date of publication of application: 04.02.2015
(73) Proprietor: L'Oréal, 75008 Paris (FR)
(72) Inventor: GENARD, Sylvie, F-92665 Asnières-sur-Seine (FR)
(74) Representative: Nony
(86) International application number: PCT/IB2013/052445
(87) International publication number: WO 2013/144869

(56) References cited:
- EP-A1- 0 876 813
- EP-A2- 1 400 237
- WO-A1-2012/137169
- WO-A1-2012/137170
- FR-A1- 2 283 223
- US-A1- 2009 022 819
- DESRUELLES F ET AL: "Clinical evaluation of a skin care programme for acne-prone very oily skin: Demonstration of the activity of a Vitreoscilla filiformis extract of the phenomena of irritation and discomfort // Evaluation clinique de l'efficacité d'un programme de soin pour peaux très grasses à tendance acnéque", NOUVELLES DERMATOLOGIQUES, STRASBOURG, FR, vol. 21, no. 3, 1 January 2002 (2002-01-01), pages 120-127, XP009091790, ISSN: 0752-5370
- GUENICHE A ET AL: "Vitreoscilla filiformis bacterium extract improves seborrheic dermatitis", JOURNAL OF INVESTIGATIVE DERMATOLOGY, NATURE PUBLISHING GROUP, GB, vol. 126, no. suppl. 3, 1 August 2006 (2006-08-01), page 99, XP009091787, ISSN: 0022-202X

## Description

The invention relates to the cosmetic use of an active agent derived from a microorganism belonging to the *Vitreoscilla* sp. genus (in particular the species: *Vitreoscilla filiformis*), as an active agent for preventing and/or treating hyperseborrhoeic conditions not associated with a dandruff condition of the scalp, which encompasses moderate to severe hyperseborrhoeic conditions not associated with a dandruff condition of the scalp.

The skin is a tissue in which the cells are joined together and integrally attached to each other. Skin tissue forms an outer coating comprising sebaceous or sweat glands, and hair follicles. The scalp, like the rest of the skin tissue, is a continually renewing epidermis where the hairs insert themselves obliquely into a deep epithelial invagination known as a hair follicle.

The scalp is rich in sebaceous glands. The sebaceous glands form, with the hair follicle, the pilosebaceous follicle.

The sebaceous glands are holocrine exocrine glands which secrete an oily substance, called sebum, originating from the breaking up of the glandular cells full of lipids. This substance consisting of cell debris and lipids and comprising approximately 58% of triglycerides, 25% of waxes, 12% of squalene and 5% of cholesterol flows directly into the pilosebaceous follicle.

The secretion of sebum is a normal phenomenon which is useful to both the skin and the head of hair. Sebum protects the scalp and gives the hair sheen by lubricating the cuticle.

Since the scalp is rich in sebaceous glands, excessive sebum secretion, or hyperseborrhoea, promotes the appearance of a moderate or severe hyperseborrhoeic condition which may be associated with difficulties, feelings of discomfort, aesthetic disorders, or even a pathological skin condition.

Hyperseborrhoea of the scalp can lead to the appearance of greasy, dull and dirty hair which has lost its natural appearance. Furthermore, the presence of an excess of sebum on the hair makes the latter lank. Hair thus made lank proves to be difficult to style, thereby representing an aesthetic drawback. Likewise, greasy hair has an increased tendency to become dirty since it readily attracts and retains dust particles in suspension in the atmosphere.

A greasy scalp represents one of the hair problems most frequently encountered, the other frequent problem being that of dry hair.

Hypersecretion of sebum, or hyperseborrhoea, may therefore lead to difficulties, feelings of discomfort, aesthetic disorders, or even a pathological skin condition. As already indicated, excessive secretion of sebum can result in a greasy, or even acneic, scalp.

Among the causes of hyperseborrhoea, mention may be made of hormonal imbalances, which can be encountered at any age in life, including during the periods of puberty and the menopause. Hormonal imbalances caused by stress, which can occur in individuals of all ages, are also known.

Difficulties associated with hyperseborrhoea of the scalp can be accentuated by the presence of certain bacterial or fungal strains on the scalp. Certain bacterial or fungal strains, such as *Propionibacterium acnes, Staphylococcus epidermis, Malassezia globosa, Malassezia restricta* and *Propionibacterium granulosum,* produce extracellular lipases on the scalp. Under the action of these lipases, the sebum triglycerides are hydrolysed to glycerol and fatty acids.

When the triglyceride concentration of the sebum decreases, for example owing to the hydrolysis of said triglycerides by bacterial lipases, the sebum fluidifies and then flows more freely along the hair, which gives the head of hair an even greasier, duller and dirtier appearance.

The excessive secretion of sebum on the scalp can also contribute to increased hair loss. In particular, hyperseborrhoea of the scalp causes the formation of plugs of sebum around and inside the hair follicles. The plugs of sebum coat the lower part of the hair bulbs responsible for hair renewal and growth, and can sometimes result in their asphyxia and generate hair loss. Hair loss, to which hyperseborrhoea of the scalp may contribute, is liable to further increase the aesthetic difficulties for the individual.

Added to that, in certain individuals, and under certain conditions, is a hyperseborrhoeic condition of the scalp which may be accompanied, at least transiently, by a dandruff condition of the scalp.

In the prior art, various solutions have been proposed for reducing excessive secretion of sebum on the scalp or for easing the consequences thereof, in particular for easing the difficulties, feelings of discomfort or aesthetic disorders which are associated therewith. Thus, many remedies have been proposed for the symptomatic treatment of the phenomena associated with excessive secretion of sebum, whether by local treatments or by systemic treatments.

In particular, cleansing agents such as abrasive agents, astringents, shampoos and soaps have been used. However, such compositions essentially have the effect of transiently removing the superficial lipids. Such compositions act exclusively by giving the scalp a less greasy appearance, without acting on the sebum hypersecretion phenomenon itself.

Local drying agents, such as sulfur, resorcinol and salicylic acid, have also been used. However, the effectiveness of such agents is principally linked to their ability to generate erythema and desquamation of the scalp, without acting on the excessive secretion of sebum.

The above prior compositions have an effect only transiently and generally result in the generation of dry hair, or even brittle hair, thereby causing other feelings of discomfort and aesthetic disorders.

According to certain studies, it has been sought to maintain a constant triglyceride concentration on the scalp by providing exogenous triglycerides which supplement the lack of endogenous triglycerides of the sebum. For example, the regular application of triglyceride-based cosmetic compositions has been described (see, in particular, United States patent No. US 4,172,149). However, such cosmetic compositions exhibited drawbacks linked to their local application, owing to the fact that the active agents diffused only very slowly to the sebaceous glands.

Other prior studies have aimed to limit the formation of lipids in the glandular cells with a view to slowing down the engorgement of the cells and preventing the breaking up of said cells, and thus reducing sebum formation. The use of acids, esters, alcohols and sulfur-containing derivatives, such as levulinic acid (see in particular Japanese patent No. JP 59 164 712), lactic acid (see in particular Belgian patent No. BE 893 182), 3,4-dihydroxyphenylalanine, zinc retinoate (see in particular European patent No. EP 38 246) or else mesulfen (see in particular European patent No. EP 19 720), has in particular been proposed.

More recently, the use of shampoos for frequent use, for example based on natural products such as citronella, tea tree oil, eucalyptus, chamomile, essence of rosemary, jojoba oil, etc., has been proposed.

There is still a need for novel active agents or novel treatments for preventing and/or treating a problem of hyperseborrhoeic conditions of the scalp.

There is still a need for a cosmetic treatment for hyperseborrhoeic conditions of the scalp that does not affect the ecoflora of the scalp, or even that reinforces the presence of a healthy ecoflora.

There is a need for cosmetic treatments for hyperseborrhoeic conditions that are capable of maintaining, or even reinforcing, the barrier properties of the scalp.

There is also a need for a cosmetic treatment for hyperseborrhoeic conditions that does not induce inflammation.

It is an object of the present invention to satisfy these needs.

Thus, the present invention aims in particular to provide a novel active agent which meets the abovementioned requirements and which in particular shows an effectiveness with regard to hyperseborrhoeic disorders of the barrier function of the scalp, in the absence of a dandruff condition of the scalp, which encompasses the moderate to severe hyperseborrhoeic disorders of the barrier function of the scalp, in the absence of a dandruff condition of the scalp.

As it is described in detail hereinafter, the novel active agent of the invention is advantageously used in the implementation of cosmetic treatment processes for hyperseborrhoeic conditions of the scalp, which encompasses moderate to severe hyperseborrhoeic conditions of the scalp, in individuals who are not affected by a dandruff condition of the scalp.

More specifically, the present invention relates to the cosmetic use of a lysate of a bacterium or bacteria belonging to the *Vitreoscilla* sp. genus (in particular species: *Vitreoscilla filiformis*), in a complete fermentation medium, as an active agent for preventing and/or treating hyperseborrhoeic conditions of the scalp not associated with a dandruff condition of the scalp, which encompasses moderate to severe hyperseborrhoeic conditions of the scalp not associated with a dandruff condition of the scalp.

For the purposes of the present invention, the expression "lysate in a complete fermentation medium" means that the lysate is used and is present, in the cosmetic or dermatological composition containing it, formulated in its complete culture medium of origin as defined hereinafter, which complete culture medium is the medium in which the bacteria were cultured until after the microbial growth phase having resulted in the use of the nutritive substrates initially present in the culture medium.

For the purposes of the present invention, the expression "complete fermentation medium" is intended to denote a medium resulting from the culturing process having been used for the growth and the cell lysis of the microorganism, said medium having undergone, moreover, no additional manipulation aimed at separating and/or removing all or part of its nonaqueous constituents.

For the purposes of the present invention, the term "preventing" means totally eliminating or partially reducing the risk of manifestation of a given phenomenon, i.e., in the present invention, the manifestation of hyperseborrhoeic conditions of the scalp not associated with a dandruff condition of the scalp, which encompasses moderate to severe hyperseborrhoeic conditions of the scalp not associated with a dandruff condition of the scalp. "Partial reduction" implies that the risk remains but to a lesser degree than before the implementation of the invention.

The term "dandruff condition" of the scalp means, according to the invention, abnormal or irregular desquamation of the cells of the stratum corneum of the scalp, resulting in the formation of large, thick clumps of cells visible to the naked eye, called "dandruff".

In certain embodiments, the present invention is aimed exclusively at preventing hyperseborrhoeic conditions of the scalp in individuals who are not affected by a dandruff condition of the scalp.

In certain embodiments, the present invention is aimed exclusively at treating hyperseborrhoeic conditions of the scalp not associated with a dandruff condition of the scalp.

A composition containing the active agent according to the invention can be administered topically.

Without wishing to be bound by what follows, the effect of the active agent according to the invention could be the result of a synergistic effect between constituents of the bacterium, which are normally separated from one another, for example its water-soluble metabolites, generated during its proliferation in its fermentation medium and conventionally present in the aqueous supernatant, and its components such as non-water soluble cell envelopes or cell envelope fractions constituting all or part of the biomass of its culture medium, or even its isolated lysate.

It has thus been shown in the examples that a cosmetic composition comprising an active agent according to the invention (a lysate of bacteria belonging to the *Vitreoscilla* sp. genus in a complete fermentation medium) has properties of reducing hyperseborrhoeic conditions of the scalp in individuals who do not have a dandruff condition of the scalp, which encompasses reducing moderate to severe hyperseborrhoeic conditions of the scalp in individuals who do not have a dandruff condition of the scalp.

It has also been shown in the examples that the effects of a cosmetic composition comprising an active agent according to the invention are long-lasting, even after a small number of applications.

The cosmetic use of a lysate of bacteria belonging to the *Vitreoscilla* sp. genus in a complete fermentation medium is therefore aimed at preventing, or else reducing or eliminating, the feelings of discomfort and the aesthetic difficulties caused by excessive secretion of sebum by the scalp, in the absence of a dandruff condition of the scalp. The feelings of discomfort encompass itching caused by the accumulation of sebum on the scalp. The aesthetic difficulties encompass a greasy scalp and greasy hair which is difficult to style.

The present invention also relates to a cosmetic process for preventing and/or treating hyperseborrhoeic conditions of the scalp not associated with a dandruff condition of the scalp, which encompasses moderate to severe hyperseborrhoeic conditions of the scalp not associated with a dandruff condition of the scalp, comprising at least one step of administering to said individual, in particular orally, an effective amount of at least one lysate of a bacterium or bacteria belonging to the *Vitreoscilla* sp. genus (in particular of the *Vitreoscilla filiformis* species) in a complete fermentation medium.

For the purposes of the present invention, the term "effective amount" means a sufficient and necessary amount of the compound under consideration for obtaining the expected effect. Such an amount may be determined by any method known to those skilled in the art, for example by means of preliminary experimental tests.

According to another of its aspects, the present invention relates to a rinse-off or leave-on cosmetic and/or dermatological composition containing, in a physiologically acceptable medium, at least one lysate of microorganism(s) belonging to the *Vitreoscilla* sp. genus (in particular of the *Vitreoscilla filiformis* species), in a complete fermentation medium.

### Complete fermentation medium

As previously stated, the expression "complete fermentation medium" is intended to denote a fermentation or else culture medium which has the same composition, at least in terms of nonaqueous constituents, or even completely, as the fermentation medium in which there was successively carried out the fermentation and the cell lysis of the microorganism devoted to forming the lysate required in parallel according to the invention.

In other words, this medium has undergone, moreover, no secondary manipulation aimed at separating and/or removing all or part of its nonaqueous constituents.

More particularly, the active agent under consideration according to the invention is formed from the lysate of microorganisms and from all or part, in terms of amount, of the culture medium having been used for the fermentation of said bacterium and in which its cell lysis was consecutively carried out (i.e. complete fermentation medium).

From the viewpoint of the aforementioned, it emerges that the active agent formed according to the invention from the lysate and from the "complete" fermentation medium contains the cytoplasmic and cytosolic fractions, the cell wall fragments and the metabolites formed and/or released during the cell lysis of said microorganism, and all of the biological entities capable of being generated and released spontaneously by the bacterium during its fermentation process and therefore already present in the fermentation medium before the cell lysis of said bacterium.

Consequently, an active agent according to the invention, i.e. formed from a lysate of a bacterium belonging to the *Vitreoscilla* sp. genus (in particular of the *Vitreoscilla filiformis* species) in a complete fermentation medium according to the invention is clearly different from the supernatant of a fermentation medium of a bacterium belonging to the *Vitreoscilla* sp. genus (in particular of the *Vitreoscilla filiformis* species).

Indeed, the active agent under consideration according to the invention, as opposed to the supernatant, contains cell fragments of said bacterium represented by the lysate.

An active agent according to the invention, i.e. formed from a lysate of a bacterium belonging to the *Vitreoscilla* sp. genus (in particular species: *Vitreoscilla filiformis)* in a complete fermentation medium according to the invention, is also different from the biomass or biomass fraction, or even from a lysate or lysate fraction, isolated from a fermentation medium of a bacterium belonging to the *Vitreoscilla* sp. genus (in particular species: *Vitreoscilla filiformis*).

Indeed, the active agent under consideration according to the invention, as opposed to this biomass or biomass fraction, or this lysate or lysate fraction, contains a significant amount of water-soluble metabolites naturally released into the culture medium during the proliferation of said bacterium.

For the purposes of the present invention, the expression "nonaqueous constituents" implies that water, which is a major constituent of conventional fermentation media, is not part of the constituents that must remain as such, i.e. in equal amount, in the complete culture medium according to the invention.

Thus, the expression "complete medium" is also understood to be a form of complete medium termed concentrated owing to the fact that it is obtained at the end of a partial evaporation of the water constituting a fermentation medium in which the culturing of the corresponding microorganism and the cell lysis thereof were consecutively carried out. Of course, this evaporation is carried out under operating conditions adjusted so as not to impair the integrity of the nonaqueous constituents forming this complete medium.

### Fermentation medium composition

By definition, a fermentation or else culture medium is a support which enables the culture and therefore, as appropriate, the growth of cells, bacteria and yeasts. In principle, the cells find in this medium the components essential for them to multiply in large number rapidly, but also sometimes elements which will make it possible to favour the growth of a specific bacterial genus or of a particular family, in this case a bacteria belonging to the *Vitreoscilla* sp. genus (in particular of the species: *Vitreoscilla filiformis*).

Its composition must therefore meet the nutritive requirements of the microorganism under consideration and necessary for the proliferation thereof.

More specifically, the composition of this culture medium must:
- cover the needs in terms of mineral ions and growth factors, and provide the carbon and energy source;
- have a pH close to the optimum pH; and
- have an optimum ionic strength (the medium may be isotonic, but this is not obligatory).

Thus, the composition of a fermentation medium suitable for the invention comprises at least:
- a carbon and energy source, generally represented by a sugar and advantageously glucose,
- a potassium and phosphorus source, like, for example, K₂HPO₄
- a nitrogen and sulfur source which can be represented by the compound (NH₄)₂SO₄,
- a magnesium source such as, for example, MgCl₂,
- a calcium source such as, for example, CaCl₂,
- an iron source, and more particularly iron citrate, the role of the citrate being to keep the iron in solution,
- a source of oligo elements chosen in particular from salts of Cu, Zn, Co, Ni, B, Ti,
- a source of water, generally sterile, essential for all forms of life,
- a pH buffer which can be represented by KH₂PO₄.

If one of these components is not present, the bacteria do not grow because they cannot by themselves compensate for its absence.

By way of illustration of a fermentation medium suitable for the growth of a microorganism in accordance with the invention, mention may particularly be made of the medium represented in Example 1 hereinafter.

An effective amount of the microorganism under consideration according to the invention is introduced therein and the whole mixture is placed under conditions suitable for the proliferation of said microorganism.

To obtain a lysate of bacteria belonging to the *Vitreoscilla* sp. genus in a complete fermentation medium, according to a method comprising a step of culturing said bacteria, those skilled in the art may refer in particular to Example 1.

Advantageously, to obtain an active agent according to the invention, i.e. a lysate of bacteria belonging to the *Vitreoscilla* sp. genus in a complete fermentation medium, the biomass (bacterial cells after the growth phase, present in the medium in which they were cultured) is frozen, for example at a temperature of -20°C, and then sterilized, preferably by heat, in particular by subjecting the previously frozen biomass to a step of heating at a temperature above 100°C. By way of illustration, the biomass sterilization step can be carried out by autoclaving, for example at a temperature of 121°C.

As emerges from the aforementioned, at the end of this culturing of said microorganism, the latter is converted into lysate form directly in the fermentation medium that was used to culture it.

### Lysis of bacteria in order to obtain an active agent according to the invention

A lysate commonly denotes a material obtained after the destruction or dissolution of biological cells via a phenomenon known as cell lysis, thus causing the release of the intracellular and cellular biological constituents naturally contained in the biological cells under consideration.

For the purposes of the present invention, the term "lysate" denotes all of the lysate obtained by lysis of the microorganism concerned, namely a bacterium belonging to the *Vitreoscilla* sp. genus (in particular of the *Vitreoscilla filiformis* species).

The lysate used is therefore formed from all of its intracellular biological constituents, in particular its metabolites and the constituents of the cell walls and membranes generated during its cell lysis.

For the purposes of the invention, the term "metabolite" denotes any substance resulting from the metabolism of the microorganism under consideration according to the invention.

This cell lysis may be accomplished via various techniques, such as, for example, an osmotic shock, a heat shock, via ultrasonication, or alternatively under a mechanical stress of centrifugation type.

More particularly, this lysate may be obtained according to the technique described in patent US 4,464,362, and especially according to the following protocol.

The fermentation medium having been used to culture the microorganism under consideration and therefore containing said microorganism is subjected to disintegration by ultrasound in order to release therein the cytoplasmic and cytosolic fractions, the cell wall fragments and the products resulting from the metabolism of this microorganism. All these components are then preserved therein in their natural distribution in a stabilized form in the "complete" fermentation medium.

Consequently, the active agent under consideration according to the invention can be obtained via a method consisting of:
- the culturing of at least one bacterium belonging to the *Vitreoscilla* sp. genus (in particular species: *Vitreoscilla filiformis*) on a fermentation medium under conditions suitable for the proliferation of said bacterium, and
- the cell lysis of said bacteria in said fermentation medium.

### Bacteria belonging to the Vitreoscilla sp. genus (in particular of the Vitreoscilla filiformis species)

As specified above, the microorganism under consideration according to the invention in the lysate form is a non-synthetic filamentous bacterium as defined in the classification of Bergey's Manual of Systematic Bacteriology (Vol. 3, sections 22 and 23, 9th edition, 1989), and belonging to the *Vitreoscilla* sp. genus (in particular species: *Vitreoscilla filiformis*).

As emerges from the examples presented hereinafter, the inventors have discovered, unexpectedly, that such a bacterium used in the form of its lysate formulated in a complete fermentation medium within the meaning of the present invention has greater properties, in terms of effectiveness, than those of the biomass obtained from the same fermentation medium.

More particularly, it is a bacterium belonging to the *Beggiatoa, Vitreoscilla, Flexithrix* or *Leucothrix* genus.

Among the bacteria that may be used, mention may be made, for example, of *Vitreoscilla filiformis* (ATCC 15551).

According to one preferred variant of the invention, it is the bacterium *Vitreoscilla filiformis.*

### Applications

As previously pointed out, the active agent under consideration according to the present invention is particularly advantageous from the viewpoint of its effectiveness with regard to certain cosmetic disorders linked to hyperseborrhoeic conditions of the scalp not associated with a dandruff condition of the scalp.

An active agent in accordance with the invention is advantageously used with a view to treating and/or preventing hyperseborrhoeic conditions and associated disorders of the scalp, with the exception of hyperseborrhoeic conditions of the scalp which are associated with a dandruff condition of the scalp, which encompasses moderate to severe hyperseborrhoeic conditions and associated disorders of the scalp, with the exception of hyperseborrhoeic conditions of the scalp which are associated with a dandruff condition of the scalp.

During hyperseborrhoeic conditions of the scalp, the skin barrier is imbalanced, and its integrity and moisturization are impaired. The skin of the scalp may be irritated and pruritic, and fragile, and may exhibit a lack of moisturization.

As emerges from the examples hereinafter, the present invention advantageously makes it possible to have a novel active agent which is particularly effective for reducing or blocking, in individuals who are not affected by a dandruff condition of the scalp, the excessive secretion of sebum which characterizes hyperseborrhoea of the scalp.

Without wishing to be bound by any theory, the Applicant thinks that the inhibition of sebum secretion, by virtue of the active agent of the invention, makes it possible to regulate this secretion in order to block the hyperseborrhoeic condition, without blocking the sebum secretion itself, which is again stabilized at a level that makes it possible to maintain the scalp-protection and hair-sheen effect.

Advantageously, an active agent in accordance with the invention is capable of reducing the risk of occurrence of side effects.

The active agent under consideration according to the invention again advantageously makes it possible to restore a healthy scalp, in perfect homoeostasis, and to re-establish a balanced ecoflora in individuals who do not have a dandruff condition of the scalp.

Consequently, the cosmetic uses, cosmetic processes and compositions according to the invention prove to be quite particularly effective in individuals who do not have a dandruff condition of the scalp, and are of use in particular for:
- preventing and/or treating hyperseborrhoeic conditions of the scalp,
- re-establishing a balanced ecoflora of the hyperseborrhoeic scalp, and/or
- preventing and/or treating the pruritus and itching associated with hyperseborrhoeic conditions of the scalp.

Generally, the present invention is directed towards a composition comprising, as sole active agent for disorders not associated with a dandruff condition of the scalp, which encompasses moderate to severe hyperseborrhoeic disorders not associated with a dandruff condition of the scalp, a lysate of a bacterium or bacteria belonging to the *Vitreoscilla* sp. genus in a complete fermentation medium.

In a particular embodiment, the cosmetic composition may comprise an additional active agent, which contributes, together with the lysate of a bacterium or bacteria belonging to the *Vitreoscilla* sp. genus in a complete fermentation medium, to solve the technical problem from the present invention.

Advantageously, such an additional cosmetic active agent can be intended to exert a cosmetic care or hygiene effect on the scalp, or even be intended to reinforce the skin barrier of the scalp associated with hyperseborrhoeic conditions, in individuals who are not affected by a dandruff condition of the scalp.

It may in particular involve a probiotic microorganism, and/or a fraction thereof, and/or a metabolite thereof, which is different from said bacterium under consideration in the active agent according to the invention and/or from the complete culture of this microorganism.

For the purposes of the present invention, the term "probiotic microorganism" means a living microorganism which, when consumed in suitable amount, has a positive effect on the health of its host (Joint FAO/WHO Expert Consultation on Evaluation of Health and Nutritional Properties of Probiotic in Food Including Powder Milk with Live Lactic Acid Bacteria, 6 October 2001), and which can in particular improve the intestinal microbial equilibrium.

According to one embodiment, a probiotic microorganism that is suitable for the invention may be chosen from *Lactobacillus* sp., *Bifidobacterium* sp., Cocci, yeasts and sporulating bacteria, and mixtures thereof.

According to one embodiment, a microorganism that is suitable for the invention is preferentially chosen from:
- (i) lactic acid bacteria: which produce lactic acid by fermentation of sugar. According to their morphology, they are divided into two groups:
   - *Lactobacillus* species: *Lactobacillus acidophilus, amylovorus, casei, rhamnosus, brevis, crispatus, delbrueckii (subsp bulgaricus, lactis), fermentum, helveticus, gallinarum, gasseri, johnsonii, plantarum, reuteri, salivarius, alimentarius, curvatus, casei subsp. casei, sake,* and
   - Cocci: *Enterococcus (faecalis, faecium), Lactococcus lactis (subsp lactis* or *cremoris), Leuconostoc mesenteroides* subsp. *dextranicum, Pediococcus acidilactici, Sporolactobacillus inulinus, Streptococcus salvarius* subsp. *thermophilus, Streptococcus thermophilus, Staphylococccus carnosus, Staphylococcus xylosus,*
- (ii) bifidobacteria or *Bifidobacterium* species: *Bifidobacterium adolescentis, animalis, bifidum, breve, lactis, longum, infantis, pseudocatenulatum,*
- (iii) yeasts: *Saccharomyces (cerevisiae* or else *boulardii),*
- (iv) other sporulating bacteria: *Bacillus (cereus var toyo* or *subtilis), Bacillus coagulans, Bacillus licheniformis, Escherichia coli* strain *nissle, Propionibacterium freudenreichii,*
- and (v) mixtures thereof.

A microorganism that is suitable for the invention may be chosen in particular from ascomycetes such as *Saccharomyces, Yarrowia, Kluyveromyces, Torulaspora, Schizosaccharomyces pombe, Debaromyces, Candida, Pichia, Aspergillus* and *Penicillium,* bacteria of the *Bifidobacterium, Bacteroides, Fusobacterium, Melissococcus, Propionibacterium, Enterococcus, Lactococcus, Staphylococcus, Peptostrepococcus, Bacillus, Pediococcus, Micrococcus, Leuconostoc, Weissella, Aerococcus, Oenococcus* and *Lactobacillus* genera, and mixtures thereof.

As other examples of probiotic microorganisms that are suitable for the invention, mention may be made of *Bifidobacterium adolescentis, Bifidobacterium animalis, Bifidobacterium bifidum, Bifidobacterium breve, Bifidobacterium lactis, Bifidobacterium longum, Bifidobacterium infantis, Bifidobacterium pseudocatenulatum, Lactobacillus acidophilus* NCFB 1748; *Lactobacillus amylovorus, Lactobacillus casei (Shirota), Lactobacillus rhamnosus* strain GG, *Lactobacillus brevis, Lactobacillus crispatus, bulgaricus, Lactobacillus delbrueckii* subsp. *lactis, Lactobacillus fermentum, Lactobacillus helveticus, Lactobacillus gallinarum, Lactobacillus gasseri, Lactobacillus johnsonii* CNCM I-1225, *Lactobacillus plantarum, Lactobacillus reuteri, Lactobacillus salivarius, Lactobacillus alimentarius, Lactobacillus curvatus, Lactobacillus casei* subsp. *casei, Lactobacillus sake, Lactococcus lactis, Enterococcus faecalis, Enterococcus faecium, Lactococcus lactis* subsp. *lactis, Lactococcus lactis* subsp. *cremoris, Leuconostoc mesenteroides* subsp. *dextranicum, Pediococcus acidilactici, Sporolactobacillus inulinus, Streptococcus salvarius* subsp. *thermophilus, Streptococcus thermophilus, Staphylococccus carnosus, Staphylococcus xylosus, Saccharomyces cerevisiae, Saccharomyces boulardii, Bacillus cereus* var. *toyo, Bacillus cereus* var. *subtilis, Bacillus coagulans, Bacillus licheniformis, Escherichia coli* strain *nissle* and *Propionibacterium freudenreichii,* and mixtures thereof.

More particularly, it may be a probiotic microorganism chosen from *Lactobacillus* sp., *Sporolactobacillus* sp., *Enterococcus* sp., *Lactococcus* sp., *Bacillus* sp., *Streptococcus* sp., *Pediococcus* sp., *Leuconostoc* sp. and *Bifidobacterium* sp., and in particular chosen from *Lactobacillus* sp. and *Bifidobacterium* sp., and mixtures thereof.

As illustrations of these probiotic microorganisms, mention may be made more particularly of *Lactobacillus johnsonii, Lactobacillus reuteri, Lactobacillus rhamnosus, Lactobacillus paracasei, Lactobacillus casei, Bifidobacterium bifidum, Bifidobacterium brave, Bifidobacterium longum, Bifidobacterium animalis, Bifidobacterium lactis, Bifidobacterium infantis, Bifidobacterium adolescentis* and *Bifidobacterium pseudocatenulatum,* and mixtures thereof.

The species that are most particularly suitable are *Lactobacillus johnsonii, Lactobacillus paracasei, Bifidobacterium adolescentis* and *Bifidobacterium longum,* which were deposited, respectively, according to the Treaty of Budapest, at the Institut Pasteur (28, rue du Docteur Roux, F-75024 Paris cedex 15) on 30/06/92, 12/01/99, 15/04/99 and 15/04/99 under the following designations CNCM I-1225, CNCM I-2116, CNCM I-2168 and CNCM I-2170, and the *Bifidobacterium lactis* (Bb 12) (ATCC27536) or *Bifidobacterium longum* (BB536) genus. The strain of *Bifidobacterium lactis* (ATCC27536) may be obtained from Hansen (Chr. Hansen A/S, 10-12 Boege Alle, P.O. Box 407, DK-2970 Hoersholm, Denmark).

Advantageously, a microorganism that is suitable for the invention, as additional active agent, may be a lactic acid probiotic microorganism.

According to one preferred embodiment, a probiotic microorganism that is suitable for the invention may in particular be a microorganism of the *Lactobacillus* sp. genus.

Preferably, a microorganism of the *Lactobacillus* sp. genus that is suitable for the invention may be chosen from the species *Lactobacillus johnsonii, Lactobacillus reuteri, Lactobacillus paracasei* and *Lactobacillus casei,* and mixtures thereof.

According to one preferred embodiment, a microorganism that is suitable for the invention may be a *Lactobacillus paracasei.*

A microorganism that is suitable for the invention may in particular be the strain *Lactobacillus paracasei* ST11 deposited according to the Treaty of Budapest, at the Institut Pasteur (28 rue du Docteur Roux, F-75024 Paris cedex 15) on 12/01/99 under the designation CNCM I-2116, and/or a fraction thereof and/or a metabolite thereof.

According to another preferred embodiment, a probiotic microorganism that is suitable for the invention may in particular be a microorganism of the *Bifidobacterium* sp. genus, and in particular *Bifidobacterium longum,* especially *Bifidobacterium longum* (BB536).

Such a microorganism may be formulated in a composition in a proportion of at least 0.0001% expressed as dry weight, in particular in a proportion from 0.0001 % to 20% and more particularly in a proportion from 0.001% to 15% by weight, in particular from 0.01% to 10% by weight and especially from 0.1% to 2% by weight relative to the total weight of the composition containing it.

### Galenical formulation

A composition containing the active agent according to the invention can be administered orally or topically.

A composition according to the invention advantageously comprises an amount of lysate of bacterium or bacteria belonging to the *Vitreoscilla* sp. genus in a complete fermentation medium ranging from 0.001% to 10% by weight relative to the total weight of dry extract of said composition. In certain embodiments, a composition according to the invention advantageously comprises an amount of lysate of bacterium or bacteria belonging to the *Vitreoscilla* sp. genus in a complete fermentation medium ranging from 0.01% to 5% by weight relative to the total weight of dry extract of said composition. In other embodiments, a composition according to the invention advantageously comprises an amount of lysate of bacterium or bacteria belonging to the *Vitreoscilla* sp. genus in a complete fermentation medium ranging from 0.1% to 1% by weight relative to the total weight of dry extract of said composition.

It can therefore be in any of the galenical forms normally available for the selected mode of administration.

The support may be of diverse nature depending on the type of composition under consideration.

As more particularly regards the compositions intended for external topical administration, they may be aqueous, aqueous-alcoholic or oily solutions, solutions or dispersions of the lotion or serum type, emulsions of liquid or semi-liquid consistency of the milk type, obtained by dispersing a fatty phase in an aqueous phase (O/W) or conversely (W/O), or suspensions or emulsions, of soft, semi-solid or solid consistency, of the cream type, aqueous or anhydrous gels, microemulsions, microcapsules, microparticles, or vesicular dispersions of ionic and/or nonionic type.

These compositions are prepared according to the usual methods.

These compositions may in particular constitute cleansing, protective, medicated or care creams, scalp care lotions, gels or foams, for instance scalp cleansing or disinfecting lotions.

They may be used for the scalp in the form of solutions, creams, gels, emulsions or foams, or alternatively in the form of aerosol compositions also containing a pressurized propellant.

A composition for topical administration according to the invention may advantageously be formulated in any galenical form that is suitable for haircare, in particular in the form of a hair lotion, a shampoo, a hair conditioner, a disentangler, a hair cream or gel, a styling lacquer, a hairsetting lotion, a treating lotion, a dye composition (in particular for oxidation dyeing) optionally in the form of a colouring shampoo, a hair-restructuring lotion, a permanent-waving composition, a lotion or gel for combating hair loss, an antiparasitic shampoo, a medicated shampoo, in particular an anti-seborrhoea shampoo, or a scalp care product, which is in particular anti-irritant, anti-ageing or restructuring, or which activates the blood circulation.

Excluded from the invention are cosmetic compositions intended for the treatment of a dandruff condition of the scalp.

When a composition of the invention is an emulsion, the proportion of the fatty phase may range from 5% to 80% by weight and preferably from 10% to 50% by weight relative to the total weight of the composition. The oils, emulsifiers and co-emulsifiers used in the composition in emulsion form are chosen from those conventionally used in the cosmetics and/or dermatology field. The emulsifier and the co-emulsifier may be present in the composition in a proportion ranging from 0.3% to 30% by weight and preferably from 0.5% to 20% by weight relative to the total weight of the composition.

When the composition of the invention is an oily solution or gel, the fatty phase may represent more than 90% of the total weight of the composition.

In a known manner, the galenical forms intended for topical administration may also contain adjuvants that are customary in the cosmetics, pharmaceutical and/or dermatology field, such as hydrophilic or lipophilic gelling agents, hydrophilic or lipophilic active agents, preserving agents, antioxidants, solvents, fragrances, fillers, screening agents, odour absorbers and colorants. The amounts of these various adjuvants are those conventionally used in the field under consideration, for example from 0.01 % to 20% of the total weight of the composition. Depending on their nature, these adjuvants may be introduced into the fatty phase and/or into the aqueous phase.

As fatty substances that may be used in the invention, mention may be made of mineral oils, for instance hydrogenated polyisobutene and liquid petroleum jelly, plant oils, for instance a liquid fraction of shea butter, sunflower oil and apricot kernel oil, animal oils, for instance perhydrosqualene, synthetic oils, in particular purcellin oil, isopropyl myristate and ethylhexyl palmitate, unsaturated fatty acids and fluoro oils, for instance perfluoropolyethers. It is also possible to use fatty alcohols, fatty acids, for instance stearic acid and, for example, waxes, in particular paraffin wax, carnauba wax and beeswax. It is also possible to use silicone compounds, for instance silicone oils and for example cyclomethicone and dimethicone, and silicone waxes, resins and gums.

As emulsifiers that may be used in the invention, mention may be made, for example, of glyceryl stearate, polysorbate 60, the mixture of cetylstearyl alcohol/cetylstearyl alcohol oxyethylenated with 33 mol of ethylene oxide sold under the name Sinnowax AO® by the company Henkel, the mixture of PEG-6/PEG-32/glycol stearate sold under the name Tefose® 63 by the company Gattefosse, PPG-3 myristyl ether, silicone emulsifiers such as cetyl dimethicone copolyol, and sorbitan monostearate or tristearate, PEG-40 stearate and oxyethylenated (20 EO) sorbitan monostearate.

As solvents that may be used in the invention, mention may be made of lower alcohols, in particular ethanol and isopropanol, and propylene glycol.

The composition of the invention may also advantageously contain a spring and/or mineral water, chosen in particular from Vittel water, waters from the Vichy Basin, and la Roche Posay water.

Hydrophilic gelling agents that may be mentioned include carboxylic polymers such as carbomer, acrylic copolymers such as acrylate/alkyl acrylate copolymers, polyacrylamides and especially the mixture of polyacrylamide, C13-14 isoparaffin and laureth-7 sold under the name Sepigel 305® by the company SEPPIC, polysaccharides, for instance cellulose derivatives such as hydroxyalkyl celluloses and in particular hydroxypropyl cellulose and hydroxyethyl cellulose, natural gums such as guar gum, locust bean gum and xanthan gum, and clays.

Lipophilic gelling agents that may be mentioned include modified clays, for instance bentones, metal salts of fatty acids, for instance aluminium stearates and hydrophobic silica, or else ethylcellulose and polyethylene.

### Cosmetic treatment process

As indicated previously, a process according to the invention can be carried out, topically, in particular by administration to the scalp of at least one lysate of a bacterium belonging to the *Vitreoscilla* sp. genus (in particular, species: *Vitreoscilla filiformis*) in a complete fermentation medium as an active agent for preventing and/or treating conditions of the scalp not associated with a dandruff condition of the scalp, and more particularly of a cosmetic composition as previously defined.

Advantageously, a process of the invention via topical administration can comprise the administration of a composition in accordance with the invention, for example in the form of cleansing creams, scalp treatment or care lotions or gels, scalp care gels or foams, for instance cleansing or disinfecting lotions, or shampoos. According to one implementation variant, the process of the invention may comprise the topical administration to the scalp of at least one active agent according to the invention, for example in the form of a shampoo, gels, sera or lotions.

A topical cosmetic process according to the invention can be carried out on a daily basis for example, at a rate of, for example, a single administration per day or one administration twice a day, for example once in the morning and once in the evening.

A topical cosmetic process according to the invention can be carried out over a time period ranging from one week to several weeks, or even several months, this period moreover possibly being repeated after periods without treatment, for several months or even several years.

By way of example, the topical administration of a compound according to the invention may be repeated, for example, 2 to 3 times a day or more and generally over an extended period of at least 4 weeks, or even 4 to 15 weeks, with, where appropriate, one or more periods of interruption.

In the description and the examples that follow, unless otherwise indicated, the percentages are percentages by weight and the ranges of values written in the form "between ... and ..." include the stated lower and upper limits.

The examples hereinafter are presented as nonlimiting illustrations of the field of the invention.

### EXAMPLES

### EXAMPLE 1 Preparation of an active agent in accordance with the invention

The complete fermentation medium is prepared by means of a culture of the *Vitreoscilla filiformis* strain, in its complete culture medium.

The initial culture medium for obtaining the complete fermentation medium has the composition described in Table 1 below.

**Table 1**

| Chemical name | [c] |
|---|---|
| Autolytic extract of yeast | 4 g/l |
| Soybean papainic peptone F | 3 g/l |
| Glucose - Roferose | 3 g/l |
| KH₂PO₄ | 0.088 g/l |
| CaCl₂ | 0.050 g/l |
| CuSO₄. 5 H₂O | 60 µg/l |
| MnSO₄. 1H₂O | 152 µg/l |
| KI | 20 µg/µl |
| ZnSO₄. 7H₂O | 200 µg/l |
| AlCl₃. 6H₂O | 100 µg/l |
| osmosed water | qs 1l |

In order to obtain a cosmetic active agent according to the invention, i.e., in this example, a lysate of *Vitreoscilla filiformis* bacteria in a complete fermentation medium, the process as described hereinafter was carried out.

The *Vitreoscilla filiformis* strain was obtained from the ATCC (strain 15551). This strain is cultured in a particular culture medium, 2BHG2, the composition of which is given above.

The biomass is obtained by continuous culture in a bioreactor with a working capacity of 3000 litres. A growth rate of approximately 70% of the µmax (µ = 0.12 H-1) is recorded during the continuous production phase. During this step, the pH (7.00), the temperature (26°C) and the dissolved oxygen (0/5%) (0 to 5 or 0.5%) are controlled. The extraction and the separation of the cells is obtained by centrifugation (10 000 g/20 min). The biomass is frozen at -20°C and is then packaged in pouches (breaking of sterility) and is stabilized by sterilization at 121°C for 30 min. The biomass is then known as Vfe.

The biomass specification analyses are the following:
- Fixed residue at 105°C (g/100 g): 4.0 to 4.5%
- total nitrogen content with respect to AM: 10.0 to 14.0%
- Microbiology: 0 microorganism/g
- 3-hydroxybutyric acid content: 2 to 10 g/l (<10 g/1)
- pH of the solution as it is: 4 to 5.

The fermentation medium is the complete culture, obtained during the continuous fermentation. The glucose in the starting medium was consumed by the microorganisms (micro-controlled by the carbon source), as were various elements of the peptones and yeast extract provided at the start. The fermentation medium currently tested is taken directly from the fermenter, and then undergoes the sterilization scheme.

The FM, which is the unconcentrated complete culture (0.7 to 0.9% of DM), is autoclaved (30 min, 121°C), as is the lysate (4.0 to 4.5% of DM).

In the examples which follow, the active agent according to the invention (the lysate of *Vitreoscilla filiformis* bacteria in a complete fermentation medium, also denoted "FM") and also a comparative active agent constituted of the isolated bacterial lysate in the absence of the complete fermentation medium were the subject of clinical tests on hyperseborrhoeic conditions, which encompasses moderate to severe hyperseborrhoeic conditions, in comparison with a reference shampoo formulation.

### EXAMPLE 2

In Example 2, the effects on hyperseborrhoeic conditions of the scalp of individuals without a dandruff condition of the scalp, which encompasses the moderate to severe hyperseborrhoeic conditions of the scalp of individuals without a dandruff condition of the scalp, respectively: (i) of a formulation of shampoo A comprising an active agent according to the invention (lysate of *Vitreoscilla filiformis* bacteria in a complete fermentation medium, prepared in accordance with Example 1) and (ii) a formulation of reference neutral shampoo B, were compared.

| Shampoo A: | weight percentages |
|---|---|
| Active agent of Example 1 | 2.04% |
| Sodium lauryl ether sulfate (2.2 ethylene oxide units) | |
| at 70% by weight in water | 21.95% |
| Cocoyl amidopropyl betaine at 38% in water | |
| (Tego Betain F50 from Evonik Goldschmidt) | 6.3% |
| Propylene glycol | 0.1% |
| 1-(Hexadecyloxy)-2-octadecanol | 1.175% |
| Cetyl alcohol | 1.325% |
| Polydimethylsiloxane 500 000 cst (MW 250 000) | 1.995% |
| Coconut acid monoisopropanolamide | 0.25% |
| Carboxyvinyl polymer (Carbopol 980 polymer from Lubrizol | 0.3% |
| Sodium chloride | 1.1% |
| Glycerol | 2% |
| Preserving agents | qs* |
| Fragrance | qs* |
| Water | qs for** 100% |

| | |
|---|---|
| * qs means "quantity sufficient" ** qs for means "quantity sufficient for" | |

| Shampoo B | weight percentage |
|---|---|
| Sodium lauryl ether sulfate (2.2 ethylene oxide units) | |
| at 70% by weight in water | 12% |
| Cocoyl betaine at 30% in water | |
| (Tego Betain AB 1214 from Evonik Goldschmidt) | 10% |
| 2-Methyl-2,4-pentanediol | 1.25% |
| Oxyethylenated lauryl alcohol (12 ethylene oxide units) | 0.25% |
| Coconut acid monoethanolamide | 0.5% |
| Sodium chloride | 1.5% |
| Glycerol | 2% |
| Preserving agents | qs* |
| Fragrance | qs* |
| Matricaria chamomilla flower hydroglycolic extract | |
| (matricaria hydroglycolic extract 7052050 from Greentech) | 1% |
| Water | qs for** 100% |

| | |
|---|---|
| * qs means "quantity sufficient" * qs for means "quantity sufficient for" | |

The lysate of bacteria in a complete fermentation medium (active agent intended for treating hyperseborrhoeic conditions with no dandruff condition according to the invention) as obtained in Example 1 is therefore used as an active agent intended for correcting hyperseborrhoeic conditions in a shampoo formulation.

### Protocol

This study was carried out in an independent study centre, on 24 healthy adult volunteer individuals who were identified following a clinical evaluation of their hyperseborrhoeic condition, which encompasses their moderate to severe hyperseborrhoeic condition, and also on the basis of an objective sebumetric measurement on the scalp.

After 1 week of wash-out with a neutral shampoo, the individuals were treated for 2 weeks, at a rate of 2 applications per week, with the shampoo containing the active agent claimed.

Objective instrumental sebumetric measurements were carried out on the scalp every week, 20 minutes, 6 hours, 24 hours and 72 hours after shampooing.

The sebumetric measurements were carried out conventionally, according to the following protocol:
- application to the scalp of a translucent test band, then
- measurement of the light transmittance properties of the test band, using a sebumetric device.

The light transmittance values measured by sebumetric measurement are converted by this apparatus into lipid quantity values. The lipid quantity values are expressed here in micrograms of lipids per square centimeter of scalp surface (µg/cm²).

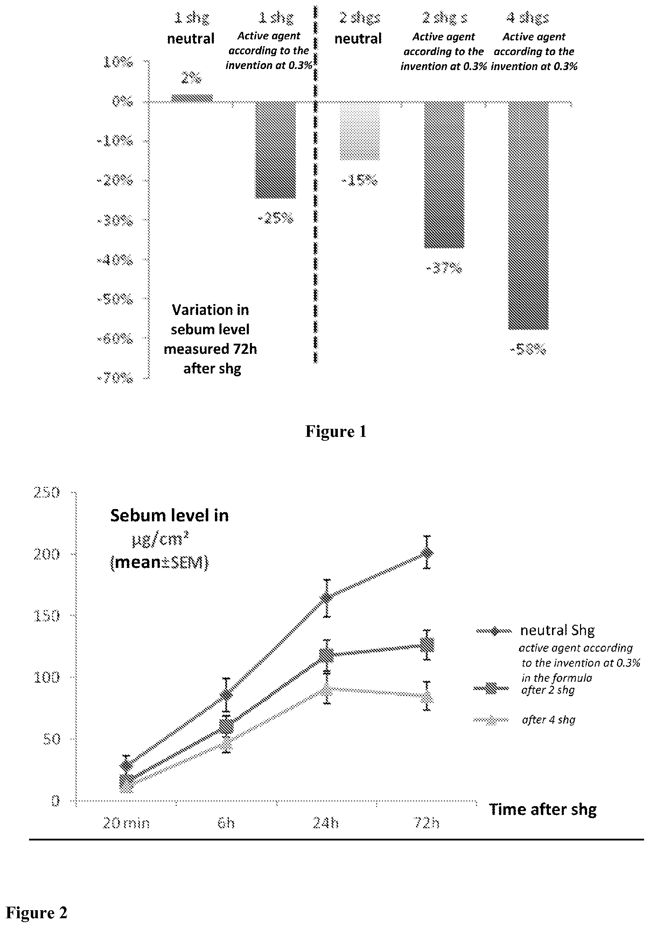

The results are given in Figures 1 and 2.
Figure 1 shows the greater decrease in sebum level with a shampoo containing the claimed active agent, in comparison with a conventional neutral shampoo (p<0.001).
Figure 2 shows that the scalp re-greasing kinetics are slower with the shampoo containing the active agent according to the invention, in comparison with a conventional neutral shampoo (p<0.001).

The results of Example 2 show, generally, the efficacy of the cosmetic active agent (lysate of *Vitreoscilla filiformis* bacteria in a complete fermentation medium, prepared in accordance with Example 1) on the excessive sebum secretion in individuals with hyperseborrhoea of the scalp (Figure 1). In particular, the results show that the inhibition of the excessive sebum secretion is indeed generated by the cosmetic active agent, and not by the other constituents of the shampoo formulation, as attested to by the results obtained with the comparative neutral shampoo formulation.

The results in Example 2 also show that the effect of inhibition of the excessive sebum secretion generated by the cosmetic active agent is long-lasting, since this inhibition effect is highly significant, even more than three days (> 72 hours) after the final shampooing operation (Figure 2).

Likewise, the results of Figure 2 show that the effect of inhibition of the excessive sebum secretion is already optimal after only two washes of the hair and of the scalp with the shampoo containing the cosmetic active agent. The inhibitory effect on the excessive sebum secretion can be further increased with a treatment comprising more than two washes of the hair and of the scalp with the shampoo containing the cosmetic active agent.

## Claims

1. Cosmetic, non-therapeutic use of a lysate of a bacterium or bacteria belonging to the *Vitreoscilla* sp. Genus, in particular species: *Vitreoscoilla filiformis*, in a complete fermentation medium, as an active agent for preventing and/or treating hyperseborrhoeic conditions of the scalp not associated with a dandruff condition of the scalp.

2. Use according to Claim 1, wherein the complete fermentation medium is all or part, in terms of amount, of the fermentation medium which has been used for the fermentation of said bacterium and in which its cell lysis was consecutively carried out.

3. Use according to any one of the preceding claims, wherein the lysate and complete fermentation medium mixture contains the cytoplasmic fractions, the cell wall fragments and the metabolites formed and/or released during the cell lysis of said bacterium and the water-soluble metabolites generated and released spontaneously by the bacterium during its fermentation process.

4. Use according to any one of the preceding claims, wherein said bacterium is the *Vitreoscilla filiformis* strain.

5. Use according to any one of the preceding claims, wherein said bacterium is the *Vitreoscilla filiformis* (ATCC 15551) strain.

6. Cosmetic, non-therapeutic process for preventing and/or treating hyperseborrhoeic conditions of the scalp of an individual, not associated with a dandruff condition of the scalp, comprising at least one step of administering to said individual at least one lysate of a bacterium or bacteria belonging to the *Vitreoscilla* sp. Genus, in particular: *Vitreoscilla filiformis* species, in a complete fermentation medium.

7. Process according to the preceding claim, wherein said bacterium is as defined in Claims 4 and 5.

8. Process according to either of Claims 6 and 7, wherein said process is carried out topically.

## Patentansprüche

1. Kosmetische, nicht therapeutische Verwendung eines Lysats von einem Bakterium oder Bakterien, welche zur *Vitreoscilla* sp. Gattung gehören, insbesondere der Art: *Vitreoscilla filiformis*, in einem kompletten Fermentationsmedium als ein Wirkstoff zum Vorbeugen und/oder Behandeln von hyperseborrhoischen Zuständen der Kopfhaut, welche nicht mit einem Zustand von Haarschuppen der Kopfhaut in Zusammenhang stehen.

2. Verwendung gemäß Anspruch 1, wobei das komplette Fermentationsmedium das gesamte oder ein Teil, bezogen auf die Menge, des Fermentationsmediums ist, welches zur Fermentation des Bakteriums verwendet wurde und in welchem seine Zelllyse nachfolgend durchgeführt wurde.

3. Verwendung gemäß einem der vorhergehenden Ansprüche, wobei das Gemisch von Lysat und komplettem Fermentationsmedium die Zytoplasmafraktionen, die Zellwandfragmente und die Metabolite, die während der Zelllyse des Bakteriums gebildet und/oder freigesetzt wurden, und die wasserlöslichen Metabolite, die spontan durch das Bakterium während seines Fermentationsverfahrens erzeugt und freigesetzt wurden, enthält.

4. Verwendung gemäß einem der vorhergehenden Ansprüche, wobei das Bakterium der Stamm *Vitreoscilla filiformis* ist.

5. Verwendung gemäß einem der vorhergehenden Ansprüche, wobei das Bakterium der Stamm *Vitreoscilla filiformis* (ATCC 15551) ist.

6. Kosmetisches, nicht therapeutisches Verfahren zum Vorbeugen und/oder Behandeln von hyperseborrhoischen Zuständen der Kopfhaut eines Individuums, welche nicht mit einem Zustand von Haarschuppen der Kopfhaut in Zusammenhang stehen, umfassend mindestens einen Schritt von Verabreichen an das Individuum mindestens eines Lysats von einem Bakterium oder Bakterien, welche zur *Vitreoscilla* sp. Gattung gehören, insbesondere der Art: *Vitreoscilla filiformis,* in einem kompletten Fermentationsmedium.

7. Verfahren gemäß dem vorhergehenden Anspruch, wobei das Bakterium wie in den Ansprüchen 4 und 5 definiert ist.

8. Verfahren gemäß einem der Ansprüche 6 und 7, wobei das Verfahren topisch durchgeführt wird.

## Revendications

1. Utilisation cosmétique non thérapeutique d'un lysat d'une bactérie ou de bactéries appartenant au genre *Vitreoscilla*, en particulier à l'espèce *Vitreoscilla filiformis*, dans un milieu de fermentation complet, en tant que principe actif pour la prévention et/ou le traitement des états d'hyperséborrhée du cuir chevelu non associés à la présence de pellicules sur le cuir chevelu.

2. Utilisation selon la revendication 1, dans laquelle le milieu de fermentation complet est totalement ou partiellement, en termes de quantité, constitué du milieu de fermentation qui a été utilisé pour la fermentation de ladite bactérie et dans lequel sa lyse cellulaire a ensuite été réalisée.

3. Utilisation selon l'une quelconque des revendications précédentes, dans laquelle le mélange de lysat et de milieu de fermentation complet contient les fractions cytoplasmiques, les fragments de paroi cellulaire et les métabolites formés et/ou libérés au cours de la lyse cellulaire de ladite bactérie et les métabolites solubles dans l'eau générés et libérés spontanément par la bactérie au cours de son processus de fermentation.

4. Utilisation selon l'une quelconque des revendications précédentes, dans laquelle ladite bactérie est la souche *Vitreoscilla filiformis.*

5. Utilisation selon l'une quelconque des revendications précédentes, dans laquelle ladite bactérie est la souche *Vitreoscilla filiformis* (ATCC 15551).

6. Procédé cosmétique non thérapeutique pour la prévention et/ou le traitement des états d'hyperséborrhée du cuir chevelu d'un individu, non associés à la présence de pellicules sur le cuir chevelu, comprenant au moins une étape d'administration audit individu d'au moins un lysat d'une bactérie ou de bactéries appartenant au genre *Vitreoscilla*, en particulier à l'espèce *Vitreoscilla filiformis*, dans un milieu de fermentation complet.

7. Procédé selon la revendication précédente, dans lequel ladite bactérie est telle que définie dans les revendications 4 et 5.

8. Procédé selon l'une ou l'autre des revendications 6 et 7, ledit procédé étant réalisé par voie topique.
